Europäisches Patentamt

European Patent Office

Office européen des brevets

(11) Publication number: **0 280 358 B1**

# EUROPEAN PATENT SPECIFICATION

(45) Date of publication of patent specification: **30.12.92** (51) Int. Cl.⁵: **A61K 39/395**

(21) Application number: **88200264.5**

(22) Date of filing: **15.02.88**

(54) **Stabilized aqueous composition containing antibodies.**

(30) Priority: **20.02.87 NL 8700422**

(43) Date of publication of application:
**31.08.88 Bulletin 88/35**

(45) Publication of the grant of the patent:
**30.12.92 Bulletin 92/53**

(84) Designated Contracting States:
**AT BE CH DE ES FR GB GR IT LI LU NL SE**

(56) References cited:
**EP-A- 0 132 488**
**EP-A- 0 170 983**
**EP-A- 0 173 648**
**WO-A-84/00890**

(73) Proprietor: **AKZO N.V.**
**Velperweg 76**
**NL-6824 BM Arnhem(NL)**

(72) Inventor: **Brinks, Gerrit Johannes**
**Van Lennephof 53**
**NL-5343 JD Oss(NL)**
Inventor: **Mentink, Maria Martina Francisca**
**Oude Wei 38**
**NL-5345 KJ Oss(NL)**

(74) Representative: **Hermans, Franciscus G.M. et al**
**Patent Department AKZO N.V. Pharma Division P.O. Box 20**
**NL-5340 BH Oss(NL)**

## Description

The present invention relates to a method of stabilizing an aqueous composition containing one or more antibodies.

Antibodies are nowadays frequently used in human and veterinary medicine for prophylactic, diagnostic or therapeutic purposes; antiviral or antibacterial antibodies are being used in the (prophylactic) treatment of infectious diseases, antigonadotrophins are frequently used to regulate hormone levels and antibodies against tumor-antigens to localize and to combat tumors.

There is therefore a great need for stable aqueous preparations which contain antibodies and which can be injected directly into human beings or animals without further operations or treatments.

Basic problem is, however, that aqueous compositions of antibodies are in practice found to be particularly unstable and no longer have any, or virtually do not have any, antibody activity even after a very short time.

An obvious approach to solving this problem might be to freeze-dry the composition concerned.

"For example, WO 84/00890 discloses a lyophilized gamma globulin concentrate into which has been included a macromolecule prior to lyophilization. During dry storage, the resulting dry compositions are stable and exhibit low anticomplement activity again without using large quantities of any one stabilizer. Before use, the lyophilized concentrate is reconstituted in sterile water to a set protein concentration for intravenous injection into a patient. This publication does not however disclose that dextran acts to stabilize monoclonal antibodies in aqueous compositions.

EP-A-173 648 discloses pharmaceutical preparations containing monoclonal antibodies for parenteral administration, such as isotonic aqueous solutions which may contain certain unidentified adjuvants such as stabilizing agents and compounds regulating the viscosity of the preparation, such as dextran, a well-known rheology modifying agent. Like WO 84/00890 however, it does not teach or suggest that dextran can solve the instability problem of monoclonal antibodies in aqueous compositions."

However, even freeze-drying of the composition has been found not to yield the result so much desired. Irreversible dimerization of the antibodies concerned during the freeze-drying process is found to be a frequently occurring secondary reaction so that even the freeze-dried compositions suffer a considerable loss in activity. In addition, dimers or higher oligomers cause anaphylactic reactions in human beings which are, of course, undesirable.

There has therefore been a search for substances which, when added to the aqueous composition of the antibodies, ensure that the biological activity and the physical quality of the antibodies remain virtually constant over a long period.

During this investigation a large number of substances was investigated but none of the substances was found to bring about the desired stability of the antibodies in question to a sufficient extent.

It has now been found that an aqueous composition of one or more antibodies remains stable over a long period of time if dextran is added to the aqueous composition.

The term dextran comprises a group of water-soluble polysaccharides having a mean molecular weight varying from approximately 20,000 to approximately 150,000 such as described, for example, in the Merck Index, 10th edition, No. 2911. Very suitable representatives within the scope of the present invention are dextrans with the relatively low molecular weight of approximately 20,000 to 75,000 such as dextran 40 having a mean molecular weight of 40,000 and dextran 70 (molecular weight 70,000).

The antibodies which are stabilized according to the present invention can, for example, be obtained from antiserum (polyclonal antibodies), or may be produced by immortalized B-lymphocytes e.g. according to the method described by Köhler and Milstein, Nature 256, 495(1975) resulting in monoclonal antibodies, or may be obtained from trioma's or quadroma's yielding bivalent monoclonal antibodies. Monoclonal antibodies obtained through recombinant DNA techniques are also specifically included.

The antibodies and more preferably the monoclonal antibodies in question may be directed against any antigen or hapten. Preferred antibodies to be used in the present invention are directed against hormones and in particular, against gonadotrophins, such as anti-human chorionic gonadotrophin (anti-hCG), anti-follicle stimulating hormones (anti-FSH), anti-luteinizing hormones (anti-LH), anti-pregnant mares serum gonadotrophin (anti-PMSG) anti-human menopausal gonadotrophin (anti-HMG).

The usual concentrations in which the antibodies are used may vary between 0.1 and about 5 mg per ml of aqueous composition and more particularly, between 0.5 and 2.5 mg/ml. The most usual concentration is, however, between 1 and 1.5 mg per ml (of the total composition).

In general a quantity of dextran is used which may vary from 0.3 to approximately 5 parts by weight of dextran per part by weight of antibody, 1 to 2 parts by weight of dextran per 1 part by weight of antibody being regarded as the most ideal.

In an absolute sense, the quantity of dextran which is used in the present invention may vary between approximately 0.05 and 20 mg/ml of aqueous composition and more particularly, between 0.5 and 5 mg per ml of aqueous composition. A quantity of dextran of approximately 1-2 mg/ml is found to be outstandingly suitable for stabilizing an aqueous composition of 1-1.5 mg of antibody. In general, dextran is not used in such quantity that is necessary to increase the viscosity of the aqueous composition significantly.

The aqueous antibody composition suitable for injection purposes can, in addition to the constituents (antibody and dextran) already mentioned, also contain other constituents such as:

- means for rendering the composition isotonic, e.g. NaCl, sorbitol, mannitol in a suitable concentration;
- means for adjusting the pH of the composition to a pH between 5.0 and 9.0 and more particularly, between 7.0 and 8.5;
- preservatives such as benzyl alcohol;
- bactericidal substances such as the parabens;
- biologically active substances such as anti-inflammatory substances, and/or anaesthetics.

The stabilized aqueous composition of, for example, monoclonal anti-gonadotrophins is prepared in the usual manner by introducing the anti-gonadotrophins concerned, dextran and any other constituents into sterilized water.

The invention is explained in more detail on the basis of the following experiment.

A solution of 1 mg per ml of monoclonal anti-hCG (mouse) in water was prepared and a certain quantity of the substance to be tested for stabilizing properties was added thereto. Each solution was kept at 4 °C, 20 °C and 40 °C for one month and then assessed for the following three factors:

(1) the presence of oligomers and, in particular, of dimers,

(2) decomposition of the anti-hCG, and

(3) physical instability, in particular the appearance of opalescence and particle formation.

In the event of a positive assessment (after storage for said month) of all three factors the solution was assigned the rating ( + ), if all three factors were negatively assessed, the rating is specified as (-), and if one or two of the above-mentioned factors was negatively assessed, the rating ("O") has been assigned.

The results obtained after storage for one month are shown in Table I.

The substances which were assessed as still positive after one month have been studied further after 3, 6 and 9 months (see Table II).

Table I

| Substance tested | Quantity | Rating (1 month) |
|---|---|---|
| phosphate buffer pH 8 | 0.07 molar | 0 |
| phosphate buffer pH 5.5 | 0.07 molar | 0 |
| NaCl | 9 mg/ml | |
| mannitol | 25 mg/ml | |
| glucose | 10 mg/ml | 0 |
| lactose | 10 mg/ml | -/0 |
| glycine | 1 mg/ml | |
| glycine | 23 mg/ml | 0/ + |
| arginine | 1 mg/ml | |
| dithioerythritol | 1 mg/ml | |
| PEG 400 | 50 mg/ml | 0 |
| sodium edetate | 1 mg/ml | + |
| benzyl alcohol | 10 mg/ml | 0 |
| mixture of methyl- and propylparaben | 1/0.2 mg/ml | 0 |
| benzylkonium chloride | 0.1 mg/ml | |
| dextran 40 | 1 mg/ml | + |
| ammonium chloride | 4.5 mg/ml | |
| albumin | 1 mg/ml | 0/ + |
| sodium carboxymethylcellulose | 5 mg/ml | 0 |

3

EP 0 280 358 B1

Table II

| Substance tested | Quantity | Rating, 3 months |
|---|---|---|
| glycine | 23 mg/ml | |
| sodium edetate | 1 mg/ml | 0 |
| albumin | 1 mg/ml | 0 |
| dextran 40 | 1 mg/ml | + |

Even after 6 and 9 months storage the rating for dextran 40 remained positive.

Example 1

The following composition was prepared for injection:

| | | |
|---|---|---|
| monoclonal anti-hCG (mouse) | 1 | mg |
| dextran 40 | 1 | mg |
| NaCl | 3.5 | mg |
| phosphate buffer pH = 8 | | |
| $Na_2HPO_4$ | 7.5 | mg |
| $NaH_2PO_4$ | 0.33 | mg |
| water for injection | to make 1 | ml |

This composition is stable for at least 9 months.

Example 2

Composition for injection consisting of:

| | | |
|---|---|---|
| monoclonal anti-hCG (mouse) | 1 | mg |
| dextran 40 | 2 | mg |
| glucose | 20 | mg |
| phosphate buffer pH = 8 | | |
| $Na_2HPO_4/NaH_2PO_4$ | 7.5 mg/0.33 | mg |
| water for injection | to make 1 | ml |

Example 3

Composition for injection consisting of:

| | |
|---|---|
| monoclonal anti-PMSG (mouse) | 1.5 mg |
| dextran 40 | 1 mg |
| benzyl alcohol | 1 mg |
| glycine buffer pH 8.5 | |
| water | to make 1 ml |

**Claims**

4

1. A method of stabilizing an aqueous composition comprising one or more monoclonal antibodies, said method comprising incorporating dextran into said aqueous composition.

2. The method according to claim 1, further characterized in that the quantity of dextran incorporated into said aqueous composition is 0.3 to 5 parts by weight per part by weight of antibody.

3. The method according to claim 1 or claim 2, further characterized in that the quantity of dextran is 1 to 2 parts by weight per part by weight of antibody.

4. The method according to claim 1, claim 2, or claim 3, further characterized in that said dextran has a mean molecular weight of between 20,000 and 75,000.

5. The method according to claim 1, claim 2, claim 3 or claim 4, further characterized in that 1 to 1.5 mg of antibody and 1 to 2 milligrams of dextran are used per milliliter of said aqueous composition.

6. The method according to claim 1, claim 2, claim 3, claim 4, or claim 5 further characterized in that the antibody is monoclonal anti-hCG.

7. The method according to claim 1, claim 2, claim 3, claim 4, or claim 5 further characterized in that the antibody is monoclonal anti-PMSG.

**Patentansprüche**

1. Verfahren zum Stabilisieren einer wässerigen Zusammensetzung, welche einen oder mehrere monoclonale Antikörper enthält, dadurch gekennzeichnet, dass der wässerigen Zusammensetzung Dextran einverleibt wird.

2. Verfahren nach Anspruch 1, dadurch gekennzeichnet, dass die Menge an Dextran, welche der wässerigen Zusammensetzung einverleibt wird, 0,3 bis 5 Gewichtsteile pro Gewichtsteil Antikörper beträgt.

3. Verfahren nach Anspruch 1 oder 2, dadurch gekennzeichnet, dass die Menge an Dextran 1 bis 2 Gewichtsteile pro Gewichtsteil Antikörper beträgt.

4. Verfahren nach Anspruch 1, 2 oder 3, dadurch gekennzeichnet, dass das Dextran ein mittleres Molekulargewicht von zwischen 20'000 und 75'000 aufweist.

5. Verfahren nach Anspruch 1, 2, 3 oder 4, dadurch gekennzeichnet, dass 1 bis 1,5 mg Antikörper und 1 bis 2 mg Dextran pro Milliliter der wässerigen Zusammensetzung verwendet werden.

6. Verfahren nach Anspruch 1, 2, 3, 4 oder 5, dadurch gekennzeichnet, dass der Antikörper monocolonales Anti-hCG ist.

7. Verfahren nach Anspruch 1, 2, 3, 4 oder 5, dadurch gekennzeichnet, dass der Antikörper monoclonales Anti-PMSG ist.

**Revendications**

1. Procédé de stabilisation d'une composition aqueuse comprenant un ou plusieurs anticorps monoclonaux, ledit procédé consistant à incorporer du dextrane à ladite composition aqueuse.

2. Procédé selon la revendication 1, caractérisé de plus par le fait que la quantité de dextrane incorporé à ladite composition aqueuse est de 0,3 à 5 parties en poids par partie en poids d'anticorps.

3. Procédé selon la revendication 1 ou la revendication 2, caractérisé de plus par le fait que la quantité de dextrane est de 1 à 2 parties en poids par partie en poids d'anticorps.

**4.** Procédé selon la revendication 1, la revendication 2 ou la revendication 3, caractérisé de plus par le fait que le poids moléculaire moyen dudit dextrane est compris entre 20 000 et 75 000.

**5.** Procédé selon la revendication 1, la revendication 2, la revendication 3 ou la revendication 4, caractérisé de plus par le fait qu'on utilise 1 à 1,5 milligramme d'anticorps et 1 à 2 milligrammes de dextrane par millilitre de ladite composition aqueuse.

**6.** Procédé selon la revendication 1, la revendication 2, la revendication 3, la revendication 4 ou la revendication 5, caractérisé de plus par le fait que l'anticorps est un anti-hCG monoclonal.

**7.** Procédé selon la revendication 1, la revendication 2, la revendication 3, la revendication 4 ou la revendication 5, caractérisé de plus par le fait que l'anticorps est un anti-PMSG monoclonal.